# NEW EUROPEAN PATENT SPECIFICATION

(11) **EP 1 693 445 B2**
(45) Date of publication and mention of the opposition decision: **08.06.2016**
(45) Mention of the grant of the patent: 02.01.2008
(21) Application number: 05425084.0
(22) Date of filing: 18.02.2005
(51) Int. Cl.: C12N 1/16, C12N 1/04, C12P 1/02, A61K 36/064, A61K 9/50

(54) **Process for the preparation of yeast microcapsules**
Verfahren zur Herstellung von Hefe-Mikrokapseln
Procédé pour la préparation de microcapsules de la levure

(43) Date of publication of application: 23.08.2006
(73) Proprietor: Gnosis S.p.A., 20033 Desio (MI) (IT)
(72) Inventor: Pagani, Hermes, 20099 Sesto San Giovanni (IT); Xaiz, Roberto, 20052 Monza (MI) (IT); Riboldi, Simona, 20035 Lissone (IT)
(74) Representative: Minoja, Fabrizio

(56) References cited:
- EP-A- 0 636 692
- EP-A- 0 862 863
- US-A- 2 523 483
- US-A- 4 643 897
- US-A- 5 665 352
- US-A- 6 159 466
- PATENT ABSTRACTS OF JAPAN vol. 1997, no. 07, 31 July 1997 (1997-07-31) & JP 09 084579 A (ASAHI CHEM IND CO LTD), 31 March 1997 (1997-03-31)

## Description

The present invention relates to the preparation of yeasts in whole, live, non-freeze-dried cells.

### Prior art

The use of freeze-dried yeast in sachets and/or capsules to treat diarrhoea is well established (US 4,643,897 and 5,665,352 filed by Biocodex, and US 5,639,496 filed by S.A.G.A.L.), and its use as a probiotic associated with selenium and/or germanium and/or chromium and/or chemotherapeutic agents, pasteurised and dried, in the form of powder, capsules, pills or tablets, is equally well known (US 6,140,107, 6,159,466, 6,197,295 and 6,368,643 filed by Viva America Marketing, Inc.).

Freeze-drying causes a shock to yeast cells which alters their integrity.

The very low temperature (freezing) and high vacuum can damage the cell walls and consequently the integrity of the cell, and can damage proteins and cell enzymes, leading to cell damage and high mortality.

Moreover, rehydration of the lyophilisate increases mortality, depending on the liquid used and the temperature, causing further damage to and weakening of the cell, with an increase in mortality and low viability in gastrointestinal transit.

### Description of the invention

The present invention allows to eliminate the above-mentioned drawbacks associated with freeze-drying, and enables whole yeast cells not subjected to the shock of freeze-drying to be obtained.

The method according to the invention, which involves drying at controlled temperature and low vacuum and subsequent microencapsulation, allows the water content of the preparation to remain unchanged over time and consequently delays aging processes, cell deterioration and contamination, leading to greater resistance to gastric acids and greater stability of the preparation over time.

The yeast biomass, prepared according to well-established fermentation techniques, is collected by centrifugation or microfiltration, washed once or twice with spring water pre-sterilised by filtration, and resuspended in sterile water to give a cell concentration of between 20 and 30 g/l of dry weight, equal to 3-6 x10¹⁰ CFU/g of dry weight. The creams thus obtained can be dried as is or, to increase their stability, can be mixed with bentonite, kaolin, silica, microcellulose or lecithinated sodium, and/or various oils such as hydrogenated coconut oil, and/or cod liver oil, etc..

The drying process must take place under vacuum at a temperature of between 20°C and 40°C, and more precisely between 25°C and 35°C, so that the internal water balance is not affected and the cells remain whole and completely viable. In orderto maintain their stability overtime, the H₂O content of the dried substance must be between 0.1 and 5%, and more precisely between 1 and 3%. The dried biomass is finely ground, and the resulting powder is then suitably mixed with excipients in common pharmaceutical use such as mannitol, lactose, saccharose, maltose, microcrystalline cellulose, silica, bentonite, kaolin, lecithinated sodium, glycerol behenate, etc.

The mixture is compressed with a tablet press (KILIAN) to produce tablets with a diameter of 15-30 mm, or more precisely 20-25 mm, and an average hardness of 20-40 Kp, and preferably 25-30 Kp. The tablets are then granulated with an oscillating granulator(VIANI) with screens having a mesh diameter of 100-3000 microns, and preferably 400-1500 microns.

The granule selected is covered with molten wax (hydrogenated vegetable fat) using a recirculating screw mixer (SAGA) and left to cool at ambient temperature, producing 500-1600 micron microcapsules containing an average of 3 (±5%) x 10⁹ CFU/g of dry weight.

The waxes used according to the invention to encapsulate the granules preferably have a melting point of between 25° and 65°C and an HLB (hydrophilia-lipophilia balance) of 4-12, and more precisely a melting point of between 30°C and 50°C and an HLB of between 5 and 10. Said waxes are advantageously selected from among a group of hydrogenated oils such as compounds of mono-, di- and triglycerides (such as BIOGAPRESS VEGETAL 8M 297 A.TO GATTEFOSSE), semi-synthetic glycerides consisting of chain C8-C 18 of saturated fatty acids of vegetable origin (such as SUPPOCIRE AM, CM, DM - GATTEFOSSE'; OSIS 60/70/80 EULI), monostearates of vegetable origin (such as GELEOL: glycerol monostearate 40-55 GATTEFOSSE'); compounds defined as mixtures of mono-, di- and triglycerides of fatty acids, mono- and diesters of polyethylene glycol (such as CELLUCIRE 44/14 and CELLUCIRE 50/13 GATTEFOSSE'), hydrogenated palm oil (such as hydrogenated vegetable fat spray 58-60 I.G.O.R.); etc:

### EXAMPLE 1

*Saccharomyces cerevisiae* DBVPG 6763 is cultivated aerobically in a medium according to Pim Van Hoek at 28°C in a laboratory fermenter (Infors AG, Techfors, Switzerland). After approx. 18h, the biomass (20 - 22 g/l of dry weight) is collected by centrifugation, washed twice with H₂O sterilised by filtration, and then resuspended in sterile H₂O to give a concentration of approx. 20% of dry weight, equal to 4-6 x 10¹⁰ CFU/g of dry weight. Bentonite 5% w/v is added to the creams. The creams thus obtained will have a concentration of approx. 25% w/v and a content of approx. 3-5 x 10¹⁰CFU/g dry weight. The creams to which the bentonite was added are dried at 32°C (±2°C) under vacuum at 5 mbars, ground to a fine powder and added to the granulate for microencapsulation as indicated below:

| | g% |
|---|---|
| *• Saccharomyces cerevisiae* powder DBVPG 6763 | 10 |
| • Mannitol | 50 |
| • Microcrystalline cellulose | 10 |
| • Colloidal silicon dioxide | 2 |
| • Mg stearate | 3 |
| • Glycerol behenate 888 | 5 |
| • Hydrogenated vegetable fat (Biogapress Vegetal 8M297, GATTEFOSSE) | 20 |

The powder is suitably mixed with all the components except for the hydrogenated vegetable fat. This mixture is used to make tablets with a 20-25 mm diameter which are then granulated in an oscillating granulator to produce 300-1500 micron granules. The granule is coated with melted vegetable fat using a recirculating screw mixer and left to cool at ambient temperature, producing 500-1600 micron microcapsules containing 3.0 (±5%) x 10⁹ CFU/g.

### EXAMPLE 2

As in example 1, wherein the formulation for microencapsulation has the following composition:

| | g% |
|---|---|
| • *Saccharomyces cerevisiae* powder DBVPG 6763 | 10 |
| • Lactose | 45 |
| • Microcrystalline cellulose | 15 |
| • Airfloat talc | 7 |
| • Mg stearate | 3 |
| • Hydrogenated vegetable fat | 20 |

The microcapsules have a content of 2.9 (±5%) x 10⁹ CFU/g.

### EXAMPLE 3

As in example 1, wherein the yeast is *Saccharomyces cerevisiae* GN/2220 - NCYC 2958.

The microcapsules have a content of 3.1 (±5%) x 10⁹ CFU/g.

### EXAMPLE 4

As in example 1, wherein the yeast is *Pichia angusta* GN/2219 *-* NCYC 2957.

The microcapsules have a content of 2.6 (±5%) x 10⁹ CFU/g.

### EXAMPLE 5

As in example 1, wherein the mannitol is at the concentration of 47% and 3% bentonite is added.

The microcapsules have a content of 3.0 (±5%) x 10⁹ CFU/g.

### EXAMPLE 6

As in example 2, wherein the lactose is at the concentration of 43% and 2% bentonite is added.

The microcapsules have a content of 2.9 (±5%) x 10⁹ CFU/g.

### EXAMPLE 7

As in example 1, wherein the mannitol is at the concentration of 44% and 6% dibasic calcium phosphate is added.

The microcapsules have a content of 3.1 (±5%) x 10⁹ CFU/g.

### EXAMPLE 8

As in example 2, wherein the lactose is at the concentration of 39% and 6% dibasic calcium phosphate is added.

The microcapsules have a content of 3.0 (±5%) x 10⁹ CFU/g.

### EXAMPLE 9

Before being covered with hydrogenated vegetable fat, the granules of *Saccharomyces* DBVPG 6763 are moistened with 10-15% cod liver oil; this is followed by the processes described in example 1. Microcapsules containing 3.2 (±5%) x 10⁹ CFU/g are obtained.

### EXAMPLE 10

The whole cells, dried as described in example 1 and ground, are moistened with cod liver oil (5-105) and microencapsulated as described in examples 1 and 2. Microcapsules containing 3.1 (±5%) x 10⁹ CFU/g are obtained.

### EXAMPLE 11

*Saccharomyces cerevisiae* DBVPG 6907 creams are obtained by proceeding as described in examples 1, 2, 5, 6, 7, 8 and 9. Microcapsules containing 2.9 - 3.4 (±5%) x 10⁹ CFU/g are obtained.

## Claims

1. Process for the preparation of probiotic yeasts in whole, live, non-freeze-dried cells in the form of microcapsules, which comprises:
a) centrifugation or microfiltration of yeast cells from the culture broth;
b) washing with water of the yeast cells and resuspension in sterile water;
c) addition to the suspension obtained in b) of bentonite, kaolin, silica, microcellulose, or lecithinated sodium, and/or natural or hydrogenated vegetable or animal oils;
d) drying at low pressure of the mixture obtained in c) at a temperature of between 20°C and 40°C to an H₂O content of between 0.5 and 5%;
e) addition of excipients to the dried substance and direct compression of the mixture;
f) granulation of the tablets obtained in e);
g) coating with molten wax of the granules obtained in f).

2. Process as claimed in claim 1, wherein the cells at stage b) are suspended in sterile water to a cell concentration of between 20 and 30 g/l of dry weight, equal to 3-6 x10¹⁰ CFU/g of dry weight.

3. Process as claimed in claim 1 or 2, wherein the yeasts are of the genus *Saccharomyces.*

4. Process as claimed in claim 3, wherein the yeast is *Saccharomyces cerevisiae.*

5. Process as claimed in any one of the above claims, wherein the drying at stage c) is performed between 25°C and 35°C.

6. Process as claimed in any one of the above claims, wherein the compression excipients are chosen from among mannitol, lactose, saccharose, maltose, microcrystalline cellulose, silica, bentonite, kaolin, lecithinated sodium and glycerol behenate.

7. Process as claimed in any one of the above claims, wherein the tablets have a diameter of 15-30 mm, and preferably 20-25 mm, and an average hardness of 20-40 Kp, and preferably 25-30 Kp.

8. Process as claimed in any one of the above claims, wherein the tablets are granulated with an oscillating granulator having screens with a mesh diameter of 100-3000 microns, and preferably 400-1500 microns.

9. Process as claimed in any one of the above claims, wherein the granules obtained from the tablets are covered with molten wax by screw mixers to produce 500-1600 micron microcapsules.

10. Process as claimed in any one of the above claims, wherein the waxes have a melting point of between 25° and 65°C and an HLB (hydrophilia-lipophilia balance) of 4-12, and preferably a melting point of between 30°C and 50°C and an HLB of between 5 and 10.

11. Process as claimed in claim 10, wherein the waxes are chosen from among hydrogenated oils, mono-, di- and triglycerides and semi-synthetic glycerides of saturated fatty acids C8-C18 of vegetable origin, monostearates of vegetable origin, mono- and diesters of polyethylene glycol, and hydrogenated palm oil.

## Patentansprüche

1. Verfahren zur Herstellung probiotischer Hefen als ganze, lebende, nicht gefriergetrocknete Zellen in Form von Mikrokapseln, welches umfasst:
a) Zentrifugation oder Mikrofiltration von Hefezellen aus dem Kulturmedium;
b) Waschen der Hefezellen mit Wasser und Resuspendieren in sterilem Wasser;
c) Zugabe von Bentonit, Kaolin, Silika, Mikrozellulose, Natriumlezithin und/oder natürlichen oder hydrierten pflanzlichen oder tierischen Ölen zu der Suspension, die in b) erhalten wurde;
d) Trocken der Mischung, die in c) erhalten wurde, bei verringertem Druck bei einer Temperatur von zwischen 20°C und 40°C zu einem Wassergehalt von zwischen 0,5 und 5%;
e) Zugabe von Bindemitteln zu der getrockneten Substanz und Direktverpressung der Mischung;
f) Granulation der Tabletten, die in e) erhalten wurden;
g) Beschichten der Granalien, die in f) erhalten wurden, mit geschmolzenem Wachs.

2. Verfahren nach Anspruch 1, wobei die Zellen in Schritt b) in sterilem Wasser zu einer Zellkonzentration von zwischen 20 und 30 g/L Trockengewicht suspendiert werden, was gleich 3-6 × 10¹⁰ CFU/g Trockengewicht ist.

3. Verfahren nach Anspruch 1 oder 2, wobei die Hefen von der Gattung *Saccharomyces* sind.

4. Verfahren nach Anspruch 3, wobei die Hefe *Saccharomyces cerevisiae* ist.

5. Verfahren nach einem der obigen Ansprüche, wobei die Trocknung in Schritt c) zwischen 25°C und 35°C durchgeführt wird.

6. Verfahren nach einem der obigen Ansprüche, wobei die Verpressungsbindemittel ausgewählt sind aus Mannitol, Laktose, Saccharose, Maltose, mikrokristalliner Zellulose, Silika, Bentonit, Kaolin, Natriumlecithin und Glycerinbehenat.

7. Verfahren nach einem der obigen Ansprüche, wobei die Tabletten einen Durchmesser von 15-30 mm, und bevorzugt von 20-25 mm, und eine durchschnittliche Härte von 20-40 Kp, und bevorzugt von 25-30 Kp, aufweisen.

8. Verfahren nach einem der obigen Ansprüche, wobei die Tabletten mit einem oszillierenden Granulator granulliert werden, der Siebe mit einem Maschendurchmesser von 100-3000 µm, und bevorzugt von 400-1500 µm, aufweist.

9. Verfahren nach einem der obigen Ansprüche, wobei die Granalien, die aus den Tabletten erhalten werden, mittels Schneckenmischern mit geschmolzenem Wachs bedeckt werden, um 500-1600 µm Mikrokapseln herzustellen.

10. Verfahren nach einem der obigen Ansprüche, wobei die Wachse einen Schmelzpunkt von zwischen 25° und 65°C und ein HLB (Hydrophilie-Lipophilie-Verhältnis) von 4-12, und bevorzugt einen Schmelzpunkt von zwischen 30°C und 50°C und ein HLB zwischen 5 und 10 aufweisen.

11. Verfahren nach Anspruch 10, wobei die Wachse ausgewählt sind aus hydrierten Ölen, Mono-, Di- und Tri-Glyceriden und semisynthetischen Glyceriden gesättigter C8-C18 Fettsäuren pflanzlichen Ursprungs, Monostearaten pflanzlichen Ursprungs, Mono- und Diestern von Polyethylenglykol und hydriertem Palmöl.

## Revendications

1. Procédé pour la préparation de levures probiotiques dans des cellules entières, vivantes et non lyophilisées sous la forme de microcapsules, qui comprend les étapes de :
a) centrifugation ou microfiltration de cellules de levure à partir du bouillon de culture ;
b) lavage avec de l'eau des cellules de levure et remise en suspension dans de l'eau stérile ;
c) addition à la suspension obtenue en b) de bentonite, de kaolin, de silice, de microcellulose, ou de sodium lécithiné, et/ou d'huiles végétales ou animales naturelles ou hydrogénées ;
d) séchage à basse pression du mélange obtenu en c) à une température comprise entre 20°C et 40°C jusqu'à une teneur en H₂O comprise entre 0,5 et 5 % ;
e) addition d'excipients à la substance séchée et compression directe du mélange ;
f) granulation des comprimés obtenus en e) ;
g) enrobage avec de la cire à l'état fondu des granulés obtenus en f).

2. Procédé selon la revendication 1, dans lequel les cellules à l'étape b) sont mises en suspension dans de l'eau stérile jusqu'à une concentration cellulaire comprise entre 20 et 30 g/l de poids sec, égale à 3 à 6 x 10¹⁰ CFU/g de poids sec.

3. Procédé selon la revendication 1 ou 2, dans lequel les levures sont du genre *Saccharomyces.*

4. Procédé selon la revendication 3, dans lequel la levure est *Saccharomyces cerevisiae.*

5. Procédé selon l'une quelconque des revendications ci-dessus, dans lequel le séchage à l'étape c) est effectué entre 25°C et 35°C.

6. Procédé selon l'une quelconque des revendications ci-dessus, dans lequel les excipients de compression sont choisis parmi du mannitol, du lactose, du saccharose, du maltose, une cellulose microcristalline, de la silice, de la bentonite, du kaolin, du sodium lécithiné et du béhénate de glycérol.

7. Procédé selon l'une quelconque des revendications ci-dessus, dans lequel les comprimés ont un diamètre de 15 à 30 mm, et de préférence de 20 à 25 mm, et une dureté moyenne de 20 à 40 Kp, et de préférence de 25 à 30 Kp.

8. Procédé selon l'une quelconque des revendications ci-dessus, dans lequel les comprimés sont granulés avec un granulateur oscillant ayant des tamis ayant un diamètre de mailles de 100 à 3 000 microns, et de préférence de 400 à 1 500 microns.

9. Procédé selon l'une quelconque des revendications ci-dessus, dans lequel les granulés obtenus à partir des comprimés sont recouverts de cire à l'état fondu à l'aide de mélangeurs à vis pour produire des microcapsules de 500 à 1 600 microns.

10. Procédé selon l'une quelconque des revendications ci-dessus, dans lequel les cires ont un point fusion compris entre 25°C et 65°C et une HLB (balance hydrophilie-lipophilie) de 4 à 12, et de préférence un point fusion compris entre 30°C et 50°C et une HLB comprise entre 5 et 10.

11. Procédé selon la revendication 10, dans lequel les cires sont choisies parmi des huiles hydrogénées, des mono-, di- et triglycérides et des glycérides semi-synthétiques d'acides gras saturés en C₈-C₁₈ d'origine végétale, des monostéarates d'origine végétale, des mono-et diesters de polyéthylèneglycol, et de l'huile de palme hydrogénée.
